Europäisches Patentamt
European Patent Office
Office européen des brevets

(19)

(11) Publication number: 0 322 034 A2

(12) EUROPEAN PATENT APPLICATION

(21) Application number: 88202880.6

(51) Int. Cl.4: C07D 413/04 , A61K 31/435

(22) Date of filing: 14.12.88

Claims for the following Contracting State: ES.

(30) Priority: 22.12.87 GB 8729924
12.05.88 GB 8811272

(43) Date of publication of application:
28.06.89 Bulletin 89/26

(84) Designated Contracting States:
AT BE CH DE ES FR GB IT LI LU NL SE

(71) Applicant: MERCK SHARP & DOHME LTD.
Hertford Road
Hoddesdon Hertfordshire EN11 9BU(GB)

(72) Inventor: Saunders, John
13 The Ridings Thorley Park
Bishop Stortford Hertfordshire(GB)
Inventor: Showell, Graham A.
5 Beehive Lane
Welwyn Garden City Hertfordshire(GB)

(74) Representative: Hesketh, Alan, Dr.
European Patent Department Merck & Co.,
Inc. Terlings Park Eastwick Road
Harlow Essex, CM20 2QR(GB)

(54) **Methyl tetrahydropyridyl oxadiazoles.**

(57) The present invention provides a compound of formula I, or a salt or prodrug thereof:

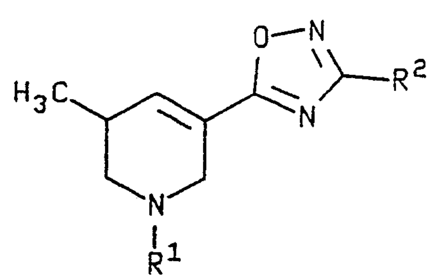

(I)

wherein $R^1$ represents hydrogen or methyl; and
$R^2$ represents hydrogen, methyl, ethyl, ethenyl, amino, methylamino, dimethylamino, chloro, bromo, cyano or methoxy; which compounds are useful in the treatment and/or prevention of neurological and mental conditions and/or of severe painful conditions.

EP 0 322 034 A2

# METHYL TETRAHYDROPYRIDYL OXADIAZOLES

The present invention relates to a class of substituted oxadiazole compounds which stimulate central muscarinic acetylcholine receptors and therefore are useful in the treatment of neurological and mental illnesses whose clinical manifestations are due to cholinergic deficiency. Such diseases include presenile and senile dementia (also known as Alzheimer's disease and senile dementia of the Alzheimer type respectively), Huntington's chorea, tardive dyskinesia, hyperkinesia, mania and Tourette Syndrome. Alzheimer's disease, the most common dementing illness, is a slowly progressive neurological disorder characterised by marked deficits in cognitive functions including memory, attention, language and visual perception capabilities. The compounds of this invention are also useful analgesic agents and therefore useful in the treatment of severe painful conditions such as rheumatism, arthritis and terminal illness.

Published European Patent Application No. 239309 discloses a class of oxadiazole compounds useful in the treatment of neurodegenerative disorders. It has now been found that certain compounds within the scope of that disclosure, but not specifically described therein, have particularly advantageous selective properties for use in the treatment of neurodegenerative diseases. The compounds of the present invention stimulate muscarinic cholinergic transmission in the cortex and thereby reverse the cholinergic deficiency, as do the whole class of compounds of EP-A-0239309, but achieve this by a mechanism selective for the cortex. That is, the present compounds do not stimulate cholinergic transmission in peripheral tissues such as heart, smooth muscle and glandular tissues. The compounds of this invention therefore have a more selective action, leading to fewer undesirable side-effects such as cardiovascular complications, diarrhoea, and salivation.

Accordingly the present invention provides a compound of formula I, or a salt or prodrug thereof:

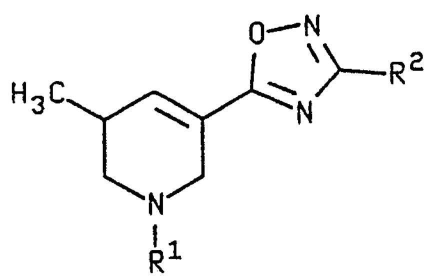

(I)

wherein $R^1$ represents hydrogen or methyl; and
$R^2$ represents hydrogen, methyl, ethyl, ethenyl, amino, methylamino, dimethylamino, chloro, bromo, cyano or methoxy.

Preferably the group $R^2$ is methyl, ethyl or dimethylamino.

The compounds of this invention have an asymmetric centre and can therefore exist as enantiomers. It is to be understood that the invention covers all such isomers and mixtures thereof.

One group of prodrugs of compounds of this invention comprises compounds of formula I wherein $R^2$ is a group which is hydrolysable in vivo to an amino group.

Groups which are hydrolysable in vivo to an amino group on the compounds of this invention may be readily ascertained by administering the compound to a human or animal and detecting, by conventional analytical techniques, the presence of the corresponding compound having an amino substituent in the urine of a human or animal. Examples of such groups include, for example, amido and urethane substituents, in particular a group of formula -NH.Q, wherein Q represents CHO, COR or $CO_2R$, and R represents an optionally substituted hydrocarbon group.

Also included within the scope of the present invention are salts of the novel compounds. It will be appreciated that salts of the compounds for use in medicine will be non-toxic pharmaceutically acceptable salts. Other salts may, however, be useful in the preparation of the compounds of the invention of their non-toxic pharmaceutically acceptable salts. Acid addition salts, for example, may be formed by mixing a solution of the compound with a solution of a pharmaceutically acceptable non-toxic acid such as hydrochloric acid, fumaric acid, maleic acid, succinic acid, acetic acid, citric acid, tartaric acid, carbonic

acid, and phosphoric acid.

Published European Patent Application No. 259621, which was published on 16th March 1988, discloses a class of compounds which overlap with the present invention when $R^1$ in formula I above represents hydrogen or methyl, and $R^2$ represents hydrogen, methyl or ethyl.

One sub-class of compounds according to the present invention comprises a compound of formula I as defined above, or a salt or prodrug thereof, wherein $R^1$ represents hydrogen or methyl; and $R^2$ represents ethenyl, amino methylamino, dimethylamino, chloro, bromo, cyano or methoxy.

Specific compounds within the scope of this invention are:
3-methyl-5-(5-methyl-1,2,4,6-tetrahydropyrid-3-yl)-1,2,4-oxadiazole;
3-methyl-5-(1,5-dimethyl-1,2,4,6-tetrahydropyrid-3-yl)-1,2,4-oxadiazole;
3-ethyl-5-(1,5-dimethyl-1,2,5,6-tetrahydropyrid-3-yl)-1,2,4-oxadiazole;
3-dimethylamino-5-(5-methyl-1,2,5,6-tetrahydropyrid-3-yl)-1,2,4-oxadiazole;
3-dimethylamino-5-(1,5-dimethyl-1,2,5,6-tetrahydropyrid-3-yl)-1,2,4-oxadiazole;
3-amino-5-(5-methyl-1,2,5,6-tetrahydropyrid-3-yl)-1,2,4-oxadiazole;
3-amino-5-(1,5-dimethyl-1,2,5,6-tetrahydropyrid-3-yl)-1,2,4-oxadiazole;
3-cyano-5-(5-methyl-1,2,5,6-tetrahydropyrid-3-yl)-1,2,4-oxadiazole;
3-cyano-5-(1,5-dimethyl-1,2,5,6-tetrahydropyrid-3-yl)-1,2,4-oxadiazole;
3-ethenyl-5-(5-methyl-1,2,5,6-tetrahydropyrid-3-yl)-1,2,4-oxadiazole;
3-ethenyl-5-(1,5-dimethyl-1,2,5,6-tetrahydropyrid-3-yl)-1,2,4-oxadiazole;
3-methoxy-5-(5-methyl-1,2,5,6-tetrahydropyrid-3-yl)-1,2,4-oxadiazole;
3-methoxy-5-(1,5-dimethyl-1,2,5,6-tetrahydropyrid-3-yl)-1,2,4-oxadiazole;
3-methylamino-5-(5-methyl-1,2,5,6-tetrahydropyrid-3-yl)-1,2,4-oxadiazole;
3-methylamino-5-(1,5-dimethyl-1,2,5,6-tetrahydropyrid-3-yl)-1,2,4-oxadiazole;
and salts and prodrugs thereof.

It is believed that the selective, non-peripheral, transmission demonstrated by the compounds of this invention is achieved by one of two alternative mechanisms. One possible mechanism is potentiation of presynaptic acetylcholine release specifically in the cortex. The compounds antagonise presynaptic receptors which are responsible for regulating the release of acetylcholine. Because such compounds fail to stimulate the receptors located in peripheral tissues, side-effects in those tissues are reduced.

Another possible mechanism involves the direct selective activation of one or more of the sub-types of muscarinic receptor located in the cortex without affecting those sub-types present in peripheral tissues leading again to compounds with fewer undesirable side-effects.

The compounds according to the invention have been found to demonstrate an affinity for the muscarinic receptor, and their tissue selectivity was assessed in three functional models, namely (i) rat superior cervical ganglion; (ii) rat atrium; and (iii) guinea pig ileum. These functional models have been described in J. Med. Chem., 1987, 30, 969. Certain of the compounds demonstrated a selective stimulant action at the ganglionic muscarinic receptor relative to the atrial and ileal receptors. Other compounds, meanwhile, appeared to antagonise the atrial and ileal sites selectively, and may therefore act by stimulating acetylcholine release in the cortex.

Since the effect of the compounds according to the invention is to stimulate acetylcholine transmission, these compounds are additionally useful as analgesic agents.

The method of treatment of this invention includes a method of treating Alzheimer's disease, senile dementia of the Alzheimer type, Huntington's chorea, tardive dyskinesia, hyperkinesia, mania or Tourette syndrome by the administration to a patient in need of such treatment of an effective amount of one or more of the novel compounds.

Moreover, the invention provides in a further aspect a method of treating severe painful conditions (e.g. rheumatism, arthritis and terminal illness) which comprises administering to a patient in need of analgesic treatment an effective amount of one or more of the analgesic compounds according to the invention.

This invention therefore also provides a pharmaceutical composition comprising a compound of the invention and a pharmaceutically acceptable carrier.

The compounds of the invention can be administered orally, parenterally or rectally at a daily dose of about 0.01 to 10 mg/kg of body weight, preferably about 0.1 to 1 mg/kg, and may be administered on a regimen of 1-4 times a day.

The pharmaceutical formulations of this invention preferably are in unit dosage forms such as tablets, pills, capsules, powders, granules, sterile parenteral solutions or suspensions, or suppositories for oral, parenteral or rectal administration. For preparing solid compositions such as tablets, the principal active ingredient is mixed with a pharmaceutical carrier, e.g. conventional tabletting ingredients such as corn starch, lactose, sucrose, sorbitol, talc, stearic acid, magnesium stearate, dicalcium phosphate or gums, and

other pharmaceutical diluents, e.g. water, to form a solid preformulation composition containing a homogeneous mixture of a compound of the present invention, or a non-toxic pharmaceutically acceptable salt thereof. When referring to these preformulation compositions as homogeneous, it is meant that the active ingredient is dispersed evenly throughout the composition so that the composition may be readily subdivided into equally effective unit dosage forms such as tablets, pills and capsules. This solid preformulation composition is then subdivided into unit dosage forms of the type described above containing from 0.1 to about 500 mg of the active ingredient of the present invention. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac, cetyl alcohol and cellulose acetate.

The liquid forms in which the novel compositions of the present invention may be incorporated for administration orally or by injection include aqueous solutions, suitably flavoured syrups and flavoured emulsions with edible oils such as cottonseed oil, sesame oil, coconut oil and peanut oil, as well as elixirs and similar pharmaceutical vehicles. Suitable dispersing or suspending agents for aqueous suspension include synthetic and natural gums such as tragacanth, acacia, alginate, dextran, sodium carboxymethylcellulose, methylcellulose, polyvinyl-pyrrolidone and gelatin.

The compounds of this invention may be prepared by a process which comprises reacting a reactive derivative of a carboxylic acid of formula II:

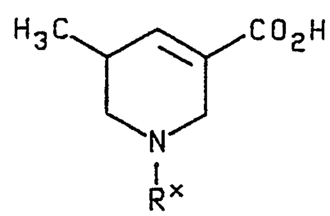

(II)

wherein $R^x$ represents the group $R^1$ or an N-protecting group; with a compound of formula III:

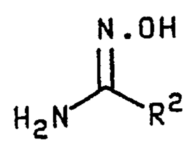

(III)

It will be appreciated that the compound of formula III can also be considered as the alternative tautomeric form IIIA:

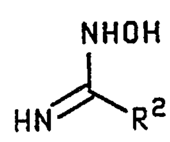

(IIIA)

Suitable reactive derivatives of the acid of formula II includes esters, for example $C_{1-4}$ alkyl esters; thioesters, for example pyridylthioesters; acid anhydrides; acid halides, for example acid chlorides; orthoesters; and primary, secondary and tertiary amides.

A preferred reactive derivative of the acid of formula II is a $C_{1-4}$ alkyl ester. The reaction is conveniently carried out in tetrahydrofuran, dimethylformamide or a lower alkanol such as ethanol, propanol or isopropanol at about 20° to 100° for about 1 to 6 hours.

The preferred N-protecting group $R^x$ is an ester group, preferably methoxycarbonyl, which may subsequently be removed by treatment with potassium hydroxide in methanol/water; or t-butoxycarbonyl, which may subsequently be removed by treatment with hydrochloric acid.

After this process is complete, a N-protecting group, if present, is removed; and the compound of formula I wherein $R^1$ represents hydrogen may be methylated, for example by treatment with methyl iodide or methyl bromide.

The starting material for the above process, i.e. the reactive derivative of an acid of formula II, may be prepared by dehydration of a compound of formula IV:

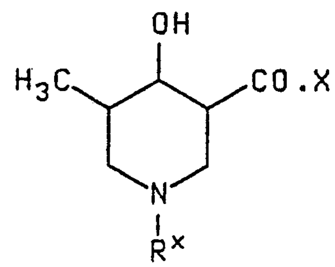

(IV)

wherein $R^x$ is as defined above, and CO.X represents a reactive derivative of a carboxylic acid. The dehydration is suitably carried out by activating the hydroxyl group by treatment with methanesulphonyl chloride in the presence of a base such as triethylamine, followed by dehydration with 1,8-diazabicyclo-[5.4.0]undec-7-ene.

The intermediate of formula IV may be prepared by a route illustrated for the compounds in which $R^x$ = $CO_2R^y$ in Scheme A or Scheme B below:

Scheme A Scheme B

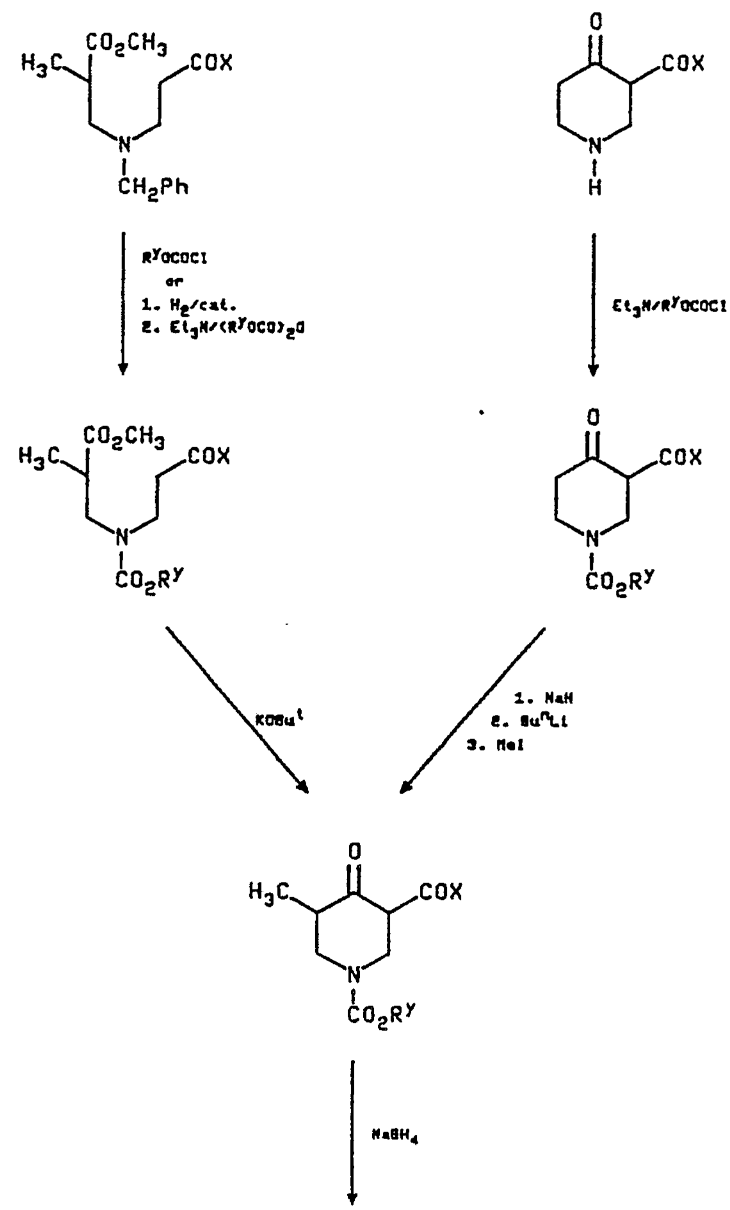


(IV)

The following Examples illustrate the preparation of compounds of this invention. The compounds according to the invention demonstrate an affinity for the muscarinic receptor, having an $IC_{50}$ (concentration required to displace 50% of specific [$^3$H]-N-methylscopolamine binding from rat cortical membrane preparations) better than 10µM. The compounds of each of the accompanying Examples demonstrate penetrability into the central nervous system, as assessed by a measurable displacement of radioligand binding using standard "ex-vivo" binding techniques (Ref: J. Neurosurg., 1985, 63, 589-592).

## EXAMPLE 1

3-Methyl-5-(5-methyl-1,2,5,6-tetrahydropyridin-3-yl)-1,2,4-oxadiazole hydrochloride

a) Methyl 2-phenylmethylaminomethyl propionate

Methyl methacrylate (214ml, 2mol), benzylamine (218ml, 2mol) and methanol (200ml) were heated under reflux for 11 hours, then left standing at room temperature for 12 hours. After the methanol had been removed the residual methyl methacrylate and benzylamine were distilled off under vacuum. The title product was distilled to afford a colourless oil (238g, 58%); bp 105-110° C (0.7mm) (bp 150-155° C (17mm), J. Med. Pharm. Chem. 1962, 5, 913); Rf = 0.30 in ethyl acetate on silica; δ (360MHz, $CDCl_3$) 1.17 (3H, d, J = 7Hz, $CHCH_3$); 2.63-2.72 (2H, m, $NCH_2CH$); 2.85-2.91 (1H, m, $CHCO_2CH_3$); 3.68 (3H, s, $CO_2CH_3$); 3.79 (2H, s, $PhCH_2N$); 7.21-7.34 (5H, m, phenyl).

b) Methyl 2-(N-2-methoxycarbonylethyl-N-phenylmethyl)aminomethyl propionate

Methyl acrylate (124ml, 1.38mol) and methyl 2-phenylmethylaminomethyl propionate (238g, 1.15mol) were heated under reflux for 24 hours then left standing at room temperture for 2 days. Distillation gave the product as a colourless oil (216.7g, 64%); bp 155-160° C (0.6mm) (bp 140°-144° C (0.8mm), U.S. patent 4,481,360 (1984)); Rf = 0.70 in ethyl acetate on silica; δ (360MHz, $CDCl_3$) 1.09 (3H, d, J = 7Hz, $CHCH_3$; 2.36-2.48 (3H, m) and 2.64-2.80 (4H, m, $NCH_2CH_2$, $NCH_2CH_2$, $NCH_2CH$ and $NCH_2CH$); 3.62 (3H, s, $CO_2CH_3$); 3.64 (2H, s, $PhCH_2N$); 3.65 (3H, s, $CO_2CH_3$); 7.19-7.32 (5H, m, phenyl).

c) Methyl 2-(N-methoxycarbonyl-N-2-methoxycarbonylethyl) aminomethyl propionate

Methyl chloroformate (172ml, 2.22mol) was added to a stirred solution of methyl 2-(N-2-methoxycarbonyl ethyl-N-phenylmethyl) aminomethyl propionate (216.7g, 0.74mol) in 1,2-dichloroethane (500ml). After heating under reflux for 8 hours the volatiles were removed in vacuo. The residue was distilled to give the title compound as a colourless oil (155.5g, 80%); bp 138-140° C (0.5mm). (Found: C, 50.86; H, 7.27; N, 5.38; $C_{11}H_{19}NO_6$ requires C, 40.57; H, 7.33; N, 5.36%); ν max (liquid film) 1735 and 1705 $cm^{-1}$ (C=O); m/e 261 ($M^+$); δ (360MHz, $CDCl_3$) 1.13 (3H, d, J = 7Hz, $CHCH_3$); 2.50-2.64 (2H, m, $CH_2CO_2CH_3$); 2.75-2.95 (1H, m, $CHCO_2CH_3$); 3.37-3.57 (4H, m, $2XNCH_2$); 3.68 (9H, s, $3XCO_2CH_3$).

d) Methyl 1-methoxycarbonyl-5-methyl-4-oxopiperidine-3-carboxylate

A solution of methyl 2-(N-methoxycarbonyl-N-2-methoxycarbonylethyl) aminomethyl propionate (54.2g, 0.21mol) in toluene (100ml) was added dropwise over 20 minutes to a stirred solution of potassium t-butoxide (23.28g, 0.21mol) in toluene (250ml) heated to reflux under a nitrogen atmosphere. After a further 1 hour at reflux the reaction mixture was cooled to room temperature then placed in an ice bath for 1 hour. The solid was collected and dissolved in water (150ml), washed with ethyl acetate (100ml) then cooled to 0° C. Dichloromethane (150ml) was added followed by concentrated hydrochloric acid (10ml) slowly and with stirring. After a further 15 minutes the organic layer was separated and the aqueous re-extracted with dichloromethane (100ml). The combined organics were washed with water (100ml), dried (sodium sulphate) then evaporated to dryness in vacuo to give the crude product as a pale yellow oil (10.0g). This oil was purified through a short silica column (Kieselgel 60H, 30g) by elution with dichloromethane to afford the title compound as a colourless oil (8.40g, 48%), pure by TLC; Rf = 0.55 on silica using ethyl acetate/petroleum ether (60-80) [1:1]. Distillation of the product yielded a colourless oil of bp 130-132° C (0.6mm). (Found: C, 51.95; H, 6.58: N, 6.32; $C_{10}H_{15}NO_5$ requires C, 52.50; H, 6.60; N, 6.11%), ν max (liquid film) 3700-3000 $cm^{-1}$ (OH, enol), 1745, 1705 and 1665 $cm^{-1}$ (C=O), 1620 $cm^{-1}$ (C=C, enol); m/e 230 $(M+H)^+$.

e) Methyl 4-hydroxy-1-methoxycarbonyl-5-methylpiperidine-3-carboxylate

Sodium borohydride (0.605g, 0.016 mol) was added portionwise to a stirred, cooled (0° C) solution of methyl 1-methoxycarbonyl-5-methyl-4-oxopiperidine-3-carboxylate (7.0g, 0.031mol) in methanol (25ml). After 30 minutes at 0° C the mixture was poured into water (25ml) and dichloromethane (40ml) was added followed by 2M hydrochloric acid (7ml). The mixture was stirred vigorously for 15 minutes then the organic layer was separated and the aqueous re-extracted with $CH_2Cl_2$ (2X40ml). The combined organics were washed with water (50ml), dried (sodium sulphate) then evaporated in vacuo to give a colourless oil (5.62g,

78%); ν max (liquid film) 3600-3150 $cm^{-1}$ (OH), 1735, 1700 and 1680 $cm^{-1}$ (C=O); m/e 231 ($M^+$).

f) Methyl 4-methanesulphonyloxy-1-methoxycarbonyl-5-methylpiperidine-3-carboxylate

Methanesulphonylchloride (1.86ml, 0.024mol) was added dropwise to a stirred, cooled (0°C) solution of the foregoing hydrox-ester (5.50g, 0.024mol) and triethylamine (3.33m, 0.024mol) in dry dichloromethane (25ml). After 1 hour water (25ml) was added and the organic layer was separated, washed with water (25ml), dried (sodium sulphate) then evaporated in vacuo to give the title compound as a pale yellow gum (6.55g, 88%); m/e 310 $(M+H)^+$.

g) Methyl 1-methoxycarbonyl-5-methyl-1,2,5,6-tetrahydropyridine-3-carboxylate

A solution of 1,8-diazabicyclo[5.4.0)undec-7-ene (2.88ml, 0.019mol) in dry dichloromethane (10ml) was added dropwise to a stirred solution of the foregoing mesylate (5.95g, 0.019mol) in dry dichloromethane (50ml). After 1 hour at room temperature 2M hydro chloric acid (30ml) was added and the mixture stirred vigorously for 10 minutes. The organic layer was separated, washed with water (30ml) then dried (sodium sulphate) and evaporated in vacuo to afford a pale yellow oil (4.40g). Purification by column chromatography on silica by elution with ethyl acetate/petroleum ether (60-80) (1:3) gave the title compound as a colourless oil which solidified on standing (1.57g, 39%), mp 51°C. (Found: C, 56.31; H, 7.10; N, 6.55; $C_{10}H_{15}NO_4$ requires C, 56.33; H, 7.09; N, 6.57%) ν max (nujol) 1715 $cm^{-1}$ (C=O), 1655 $cm^{-1}$ (C=C); m/e 213 ($M^+$); δ (360MHz, $CDCl_3$) 1.07 (3H, d, J=7Hz, $CHCH_3$); 2.46-2.56 (1H, m, 5-CH); 2.84-2.94 (1H, m, 6-CH); 3.73 (3H, s) and 3.76 (3H, s, $2XCOOCH_3$); 3.80-3.90 (1H, m, 6-CH); 3.92-4.01 (1H, m) and 4.25-4.33 (1H, m, 2X2-CH); 6.92 (1H, broad 2, 4-CH).

h) 3-Methyl-5-(1-methoxycarbonyl-5-methyl-1,2,5,6-tetrahydropyridin-3-yl)-1,2,4-oxadiazole

Acetamide oxime (1.21g, 0.016mol) was stirred in dry tetrahydrofuran (35ml) with 4A molecular sieves (7g) under a nitrogen atmosphere for 1 hour. Sodium hydride (0.698g of a 55% dispersion in oil) was added in portions and the reaction mixture was stirred at 50°C for 1 hour. A solution of methyl 1-methoxycarbonyl-5-methyl-1,2,5,6-tetrahydropyridine-3-carboxylate (1.40g) in dry tetrahydrofuran (20ml, pre-dried by stirring over 4A molecular sieves (3g) for 30 minutes) was added and the reaction mixture was stirred whilst heating under reflux for 2 hours. The reaction mixture was cooled, water (50ml) was added followed by dichloromethane (50ml). After stirring for 15 minutes the mixture was filtered through hyflo then the organic layer was separated and the aqueous re-extracted with $CH_2Cl_2$ (50ml). The combined organics were dried (sodium sulphate) then evaporated do dryness in vacuo to give the crude product which was purified by column chromatography on silica by elution with dichloromethane/methanol (200:1) yielding the title product as a colourless oil which crystallised on standing (1.03g, 66%); mp 64°C. (Found: C, 55.57; H, 6.36; N, 17.54; $C_{11}H_{15}N_3O_3$ requires C, 55.68; H, 6.37; N, 17.71%); ν max (liquid film) 1710 $cm^{-1}$ (C=O), 1655 $cm^{-1}$ (C=N); m/e 238 $(M+H)^+$; δ (360MHz, $CDCl_3$) 1.13 (3H, d, J = 7Hz, $CHCH_3$); 2.41 (3H, s, oxadiazole-$CH_3$); 2.56-2.66 (1H, m, 5-CH); 2.94-3.02 (1H, m, 6-CH); 3.75 (3H, s, $COOCH_3$); 3.82-4.00 (1H, m, 6-CH); 4.19 (1H, d, J = 18Hz, 2-CH); 4.51 (1H, dm, J = 18Hz, 2-CH); 6.99 (1H, m, 4-CH).

i) 3-Methyl-5-(5-Methyl-1,2,5,6-tetrahydropyridin-3-yl)-1,2,4-oxadiazole hydrochloride.

3-Methyl-5-(1-methoxycarbonyl-5-methyl-1,2,5,6-tetrahydropyridin-3-yl)-1,2,4-oxadiazole (0.75g, 0.003 mol) and potassium hydroxide (0.18 g, 0.0032 mol) were heated under reflux, with stirring, in methanol (4 ml) and water (4 ml) for 9 hours. The reaction mixture was evaporated to dryness and the residue partitioned between water (3 ml) and dichloromethane (20 ml). The organic layer was separated, aqueous re-extracted with dichloromethane (2 x 20 ml). The combined organics were dried then evaporated to dryness in vacuo to give a pale yellow oil which was purified by column chromatography on silica using dichloromethane/methanol (gradient elution, 200:1 to 25:1), to afford the title compound free base as a colourless oil (0.135g, 25%). The hydrochloride salt had mp 125-126°C (propan-2-ol/diethyl ether); Rf = 0.40 in dichloromethane/methanol (9:1) on silica; ν max (nujol) 2800 - 2500 $cm^{-1}$ ($NH^+$), 1660 $cm^{-1}$ (C=N); δ (360MHz, $CDCl_3$) 1.24 (3H, d, J = 7Hz, $CHCH_3$); 2.41 (3H, s, oxadiazole- $CH_3$); 2.95 - 3.05 (2H, m, 5-CH

and 6-CH); 3.60 -3.67 (1H, m, 6-CH); 4.09 (1H, dm, J = 16Hz, 2-CH); 4.14 (1H, dm, J=16Hz, 2-CH); 7.20 (1H, d, J= 2Hz, 4-CH). (Found: C, 49.58; H, 6.51; N, 18.88; $C_9H_{13}N_3O.HCl.0.2H_2O$ requires C, 49.29; H, 6.62; N, 19.16%); m/e 179 ($M^+$ of free base).

## EXAMPLE 2

### 3-Ethyl-5-(5-methyl-1,2,5,6-tetrahydropyridin-3-yl)-1,2,4-oxadiazole hydrogen oxalate

#### a) Methyl 2-(2-methoxycarbonylethyl) aminomethyl propionate hydrochloride

Methyl 2-(N-2-methoxycarbonylethyl-N-phenyl methyl)aminomethyl propionate (300 g, 1.02 mol, prepared as in Example 1b) was hydrogenated at 3.5x $10^5 N.m^{-2}$ (50 psi) over 20% palladium hydroxide on carbon (3 g) in methanol (100 ml) and 5M hydrochloric acid (210 ml) for 6 hours. The reaction mixture was filtered through hyflo, and the aqueous layer separated. The organic layer was extracted with water (200 ml) then the combined aqueous layers were evaporated to dryness in vacuo then the residue was dried over phosphorous pentoxide for 3 days to give a colourless gum (235 g, 79%); RF = 0.80 in dichloromethane/methanol (9:1) on neutral alumina. (Found: C, 43.34; H, 7.35; H, 5.90; $C_9H_{17}NO_4$. $HCl.H_2O$ requires C, 43.46; H, 7.70; N, 5.63%); $\delta$ (360MHz, $D_2O$) 1.27 (3H, d, J = 7Hz, $CHCH_3$); 2.80 - 2.93 (2H, m, $CH_2CO_2CH_3$); 3.00 - 3.04 (1H, m, $CHCO_2CH_3$); 3.14 - 3.22 (1H, m, $NCH_2CH$); 3.31 -3.42 (3H, m, $NCH_2CH$, $NCH_2CH_2$); 3.76 (3H, s, $CO_2CH_3$); 3.77 (3H, s, $CO_2CH_3$).

#### b) Methyl 2-(N-t-butyloxycarbonyl-N-2-methoxycarbonylethyl)aminomethyl propionate

Triethylamine (272 ml, 1.96 mol) was added dropwise to a stirred suspension of methyl 2-(2-methoxycarbonylethyl)aminomethyl propionate hydrochloride (234 g, 0.98 mol) in dry dichloromethane (1.5 litres). After 15 minutes di-t-butyldicarbonate (285 g, 1.30 mol) was added dropwise over 40 minutes. After 18 hours at room temperature the mixture was filtered, washed with 0.5M hydrochloric acid (2 x 500 ml), water (2 x 500 ml), saturated sodium hydrogen carbonate (500 ml) then dried (sodium sulphate) and evaporated to dryness in vacuo to give a colourless oil which was distilled under vacuum (151.5 g, 51%); bp 155 - 158° C (2 mm); Rf = 0.65 in ethyl acetate on silica. (Found: C, 55.68; H, 8.32; N, 4.60; $C_{14}H_{25}NO_6$ requires C, 55.43; H, 8.31; 4.62%); $\nu$ max (liquid film) 1740 and 1700 $cm^{-1}$ (C = O); m/e 304 $(M + H)^+$, (Found: $(M + H)^+$, 304.1747; $C_{14}H_{26}NO_6$ requires (M + H), 304.1760); $\delta$ (360MHz, $CDCl_3$) 1.13 (3H, d, J = 7Hz, $CHCH_3$); 1.45 (9H, s, $(CH_3)_3C$); 2.50 - 2.62 (2H, m, $CH_2CO_2CH_3$); 2.72 - 2.90 (1H, m, $CHCO_2CH_3$); 3.28 - 3.56 (4H, m, 2 x $NCH_2$); 3.68 (6H, s, 2 x $CO_2CH_3$).

#### c) Methyl 1-t-butyloxycarbonyl-5-methyl-4-oxopiperidine-3-carboxylate

A solution of methyl 2-(N-t-butyloxycarbonyl-N-2-methoxycarbonylethyl) aminomethyl propionate (30 g, 0.099 mol) in toluene (30 ml) was added dropwise over 20 minutes to a stirred solution of potassium -t-butoxide (11.1 g, 0.099 mol) in toluene (100 ml) at 80° C (oil bath temperture) under a nitrogen atmosphere. After a further 1 hour at 80° C the reaction mixture was cooled to room temperature then placed in an ice bath for 1 hour. The solid was filtered off, washed with ethyl acetate (100 ml), then dissolved in water (100 ml). Dichloromethane (100 ml) was added and the aqueous was acidified to pH ~4 with concentrated hydrochloric acid whilst vigorously stirring. The organic layer was separated, aqueous re-extracted with dichloromethane (100 ml). The combined organics were washed with water (100 ml), dried (sodium sulphate) then evaporated to dryness to give a colourless oil (20 g). This oil was purified through a short silica column (Kieselgel 60H, 40 g) by elution with dichloromethane to afford the title compound as a colourless oil (17.8 g, 66%), pure by TLC; Rf = 0.70 in ethyl acetate/petroleum ether (60-80) [1:1] on silica. Distillation of the product yielded a colourless oil of bp 120 - 122° C (0.2 mm). (Found: C, 56.96, H, 7.72; N, 5.05; $C_{13}H_{21}NO_5$ requires C, 57.55; H, 7.80; N, 5.16%, $\nu$ max (liquid film) 3300 - 2700 $cm^{-1}$ (OH, enol), 1750, 1700 and 1665 $cm^{-1}$ (C = O), 1620 $cm^{-1}$ (C = C, enol); m/e 214 $(M - C(CH_3)_3)^+$.

d) Methyl 1-t-butyloxycarbonyl-4-hydroxy-5-methylpiperidine-3-carboxylate

Sodium borohydride (0.98 g, 0.026 mol) was added portionwise to a stirred, cooled (0°C) solution of methyl 1-t-butyloxycarbonyl-5-methyl-4-oxopiperidine-3-carboxylate (14.0 g, 0.052 mol) in methanol (30 ml). After 30 minutes at 0°C the mixture was poured into water (30 mol) then dichloromethane (40 ml) was added followed by 2M hydrochloric acid dropwise to pH~4. After stirring vigorously for 15 minutes the organic layer was separated and the aqueous re-extracted with dichloromethane (2 x 40 ml). The combined organics were washed with water (40 ml), dried (sodium sulphate) then evaporated in vacuo to give a colourless oil (14.05 g, 99%); ν max (liquid film) 3600 - 3400 $cm^{-1}$ (OH), 1740, 1680 and 1660 $cm^{-1}$ (C = O); m/e 274 $(M + H)^+$.

e) Methyl 1-t-butyloxycarbonyl-4-methanesulphonyloxy-5-methylpiperidine-3-carboxylate

Methanesulphonyl chloride (3.9 ml, 0.050 mol) was added dropwise to a stirred, cooled (0°C) solution of the foregoing hydroxy-ester (13.80 g, 0.050 mol) and triethylamine (6.9 ml, 0.050 mol) in dry dichloromethane (50 ml). After 2 hours water (50 ml) was added and the organic layer was separated, washed with water (50 ml), dried (sodium sulphate) then evaporated in vacuo to give the title compound as a yellow gum (15.5 g, 88%); m/e 352 $(M + H)^+$.

f) Methyl 1-t-butyloxycarbonyl-5-methyl-1,2,5,6-tetrahydropyridine-3-carboxylate

A solution of 1,8-diazabicyclo[5.4.0]undex-7-ene (6.6 ml, 0.044 mol) in dry dichloromethane (20 ml). After 2 hours at room temperature water (50 ml) was added and the mixture stirred for 15 minutes. The organic layer was separated, aqueous re-extracted with dichloromethane (50 ml), then the combined organics were washed with 0.5M hydrochloric acid (50 ml), water (50 ml), dried (sodium sulphate) and evaporated to dryness in vacuo to give a yellow gum (12.20 g). Purification by column chromatography on silica by elution with dichloromethane/methanol (100:1) afforded the title compound as a pale yellow oil (5.72 g, 51%), bp 115-116°C (0.2 mm). Rf = 0.65 in ethyl acetate on silica. (Found: C, 56.87; H, 8.07; N, 4.98; $C_{13}H_{21}NO_4.H_2O$ requires C, 57.12; H, 8.48; N, 5.12%); νmax (liquid film) 1710 and 1695 $cm^{-1}$ (C = O), 1655 $cm^{-1}$ (C = C); m/e 256 $(M + H)^+$, (Found: $(M + H)^+$, 256.1527; $C_{13}H_{22}NO_4$ requires (M +H), 256.1549); δ (360MHz, $d_6$DMSO) 0.99 (3H, d, J = 7Hz, $CHCH_3$); 1.41 (9H, s, $C(CH_3)_3$); 2.38 - 2.56 (1H, m, 5-CH); 2.80 - 3.10 (1H, m, 6-CH); 3.57 - 3.63 (1H, m, 6-CH); 3.96 (3H, s, $CO_2CH_3$); 3.93 (1H, dm, J = 18Hz, 2-CH); 4.00 - 4.08 (1H, m, 2-CH); 6.84 - 6.87 (1H, m, 4-CH).

g) 3-Ethyl-5-(1-t-butyloxycarbonyl-5-methyl-1,2,5,6-tetrahydropyridin-3-yl)-1,2,4-oxadiazole

Propionamide oxime (1.72 g, 0.0195 mol) was stirred in dry tetrahydrofuran (40 ml) with 4A molecular sieves (12 g) for 1 hour under a nitrogen atmosphere. Sodium hydride (0.85 g of a 55% dispersion in oil, 0.0195 mol) was added in portions and the reaction mixture was stirred at 50°C for 1 hour. A solution of methyl 1-t-butyloxycarbonyl-5-methyl-1,2,5,6-tetrahydropyridine-3-carboxylate (2.0 g, 0.0078 mol) in dry tetrahydrofuran (40 ml, pre-dried by stirring over 4A molecular sieves (8 g)) was added and the reaction mixture was stirred whilst heating under reflux for 2 hours. The reaction mixture was cooled, water (80 ml) was added followed by dichloromethane (80 ml). After stirring for 15 minutes the mixture was filtered through hyflo, the organic layer was separated, and the aqueous re-extracted with dichloromethane (80 ml). The combined organics were washed with 0.5M hydrochloric acid (50 ml), water (50 ml), dried (sodium sulphate) then evaporated to dryness in vacuo to give the crude product which was purified by column chromatography on silica by elution with ethyl acetate/petroleum ether (60 - 80) [1:4] to afford the title product as a colourless oil (1.15 g, 50%). Rf = 0.63 in ethyl acetate/petroleum ether (60 - 80) [1:1] on silica. (Found: C, 64.31; H, 8.58; N, 12.53; $C_{15}H_{23}N_3O_3.0.5C_6H_{14}$ requires C, 64.26; H, 8.98; N, 12,49%); νmax (liquid film) 1705 $cm^{-1}$ (C = O), 1660 $cm^{-1}$ (C = C, C= N); m/e 293 $(M^+)$ (Found: $M^+$, 293.1736; $C_{15}H_{23}N_3O_3$ requires M, 293.1739); δ (360MHz, $CDCl_3$) 1.11 (3H, d, J = 7Hz, $CHCH_3$); 1.33 (3H, t, J = 8Hz, $CH_2CH_3$); 1.49 (9H, s, $C(CH_3)_3$); 2.52 - 2.64 (1H, m, 5-CH); 2.76 (2H, q, J=8Hz, $CH_2CH_3$); 2.86 - 3.08 (1H, m, 6-CH); 3.70 - 3.96 (1H, m, 6-CH); 4.10 - 4.26 (1H, m, 2-CH); 4.28 (1H, dm, J = 18Hz, 2-CH); 6.96 -7.02 (1H, m, 4-CH).

h) 3-Ethyl-5-(5-methyl-1,2,5,6-tetrahydropyridin-3-yl)-1,2,4-oxadiazole hydrogen oxalate

The foregoing oxadiazole (1.05 g, 0.0036 mol) was dissolved in dry dichloromethane (10 ml). Trifluroacetic acid (1.65 ml, 0.021 mol) was added and the solution stirred at room temperature for 24 hours. The solution was evaporated to dryness, the residue was dissolved in water (10 ml), basified to pH = 10 with potassium carbonate and extracted with dichloromethane (3 x 20 ml). The combined organics were dried (sodium sulphate) then evaporated to dryness in vacuo to afford the title compound free base as a pale yellow oil (0.66 g, 96%), pure by TLC, Rf = 0.44 in dichloromethane/methanol (9:1) on silica. The hydrogen oxalite salt had mp 128 - 130 °C (propan-2-ol/methanol (3:1)). (Found: C, 50.69; H, 6.00; N, 14.72; $C_{10}H_{15}N_3O$. $C_2H_2O_4$ requires C, 50.88; H, 6.05; N, 14.83%); ν max (nujol) 3600 - 3100 $cm^{-1}$ (OH of oxalic acid), 2800 - 2400 $cm^{-1}$ ($NH^+$), 1735 $cm^{-1}$ (C = O of oxalic acid), 1660 $cm^{-1}$ (C = C, C= N and C = O of oxalic acid); m/e 193 ($M^+$ of free base); δ (360 MHz, $D_2O$) 1.22 (3H, d, J = 7Hz, $CHCH_3$); 1.28 (3H, t, J = 7.5 Hz, $CH_2CH_3$); 2.78 (2H, q, J = 7.5 Hz, $CH_2CH_3$); 2.90 - 3.04 (2H, m, 5-CH and 6-CH); 3.58 - 3.66 (1H, m, 6-CH); 4.09 (1H, dt, J = 3, 16 Hz, 2-CH); 4.13 (1H, d, J = 16 Hz, 2-CH); 7.20 (1H, broad s, 4-CH).

## EXAMPLE 3

3-Dimethylamino-5-(5-methyl-1,2,5,6-tetrahydropyridin-3-yl)-1,2,4-oxadiazole hydrochloride

a) Methyl 1-t-butyloxycarbonyl-5-methyl-1,2,5,6-tetrahydropyridine-3-carboxylate

To a stirred solution of methyl 1-t-butyloxycarbonyl-4-hydroxy-5-methylpiperidine-3-carboxylate (28.85 g, 0.11 mol, prepared as in Example 2d) in dry dichloromethane (80 ml) was added triethylamine (30 ml, 0.22 mol) dropwise, followed by methanesulphonyl chloride (17 ml, 0.22 mol) dropwise at such a rate to maintain gentle reflux. After addition the reaction mixture was stirred at room temperature for 4 hours. 1,8-Diazabicyclo[5.4.0]undex-7-ene (33 ml, 0.22 mol) was added dropwise and the reaction mixture was stirred at room temperature for 6 hours. Water (100 ml) was added followed by further dichloromethane (50 ml). After stirring for 15 minutes the organic layer was separated, aqueous re-extracted with dichloromethane (2 x 100 ml). The combined organics were washed with 0.5M hydrochloric acid (100 ml), water (100 ml) then dried (sodium sulphate) and evaporated in vacuo to give a red oil (31.6 g) which was purified by column chromatography on silica gel by elution with dichloromethane/methanol (100:1) to afford the title compound as a pale yellow oil (13.0 g, 46%) which had identical spectroscopic and chromatographic properties as the product obtained in Example 2f.

b) 3-Dimethylamino-5-(1-t-butyloxycarbonyl-5-methyl-1,2,5,6-tetrahydropyridin-3-yl)-1,2,4-oxadiazole

The title compound was prepared from N,N-dimethylhydroxyguanidine hydrochloride (2.02 g, 0.0145 mol) and methyl 1-t-butyloxycarbonyl-5-methyl-1,2,5,6-tetrahydropyridine-3-carboxylate (1.50 g, 0.0059 mol) in a manner similar to that described in Example 2g. The product was isolated as a pale yellow oil (1.24 g, 68%) which solidified on standing, mp 35 - 35 °C; Rf = 0.70 in ethyl acetate on silica; ν max (liquid film) 1700 $cm^{-1}$ (C = O), 1660 $cm^{-1}$ (C = C, C = N); m/e 308 ($M^+$), (Found: $M^+$, 308.1832; $C_{15}H_{24}N_4O_3$ requires M, 308.1848); δ (360 MHz, $CDCl_3$) 1.10 (3H, d, J = 7 Hz, $CHCH_3$); 1.49 (9H, s, $C(CH_3)_3$); 2.50 - 2.62 (1H, m, 5-CH); 2.84 - 3.10 (1H, m, 6-CH); 3.01 (6H, s, $N(CH_3)_2$); 3.64 - 3.86 (1H, m, 6-CH); 4.04 -4.22 (1H, m, 2-CH); 4.37 (1H, dm, J = 18 Hz, 2-CH); 6.86 - 6.90 (1H, m, 4-CH).

c) 3-Dimethylamino-5-(5-methyl-1,2,5,6-tetrahydropyridin-3-yl)-1,2,4-oxadiazole hydrochloride

The title compound free base was obtained from the foregoing oxadiazole (1.20 g, 0.0039 mol) as a pale yellow oil (750 mg, 92%) by the procedure described for Example 2h. The hydrochloride salt had mp 207 - 209 °C (propan-2-ol/diethyl ether), Rf = 0.35in dichloromethane/methanol (9:1) on silica. (Found: C, 48.54; H, 6.97; N, 22.45; $C_{10}H_{16}N_4O$. HCl.0.25H2) required C, 48.20; H, 7.07; N, 22.48%); ν max (nujol) 2800-2400 $cm^{-1}$ ($NH^+$), 1660 $cm^{-1}$ (C = C, C = N); m/e 209 $(M + H)^+$ of free base, δ (360 MHz, $D_2O$)

1.23 (3H, d, J = 7 Hz, $CHCH_3$); 2.92 - 3.03 (2H, m, 5-CH and 6-CH); 3.00 (6H, s, $N(CH_3)_2$); 3.58 - 3.65 (1H, m, 6-CH); 4.06 (1H, dm, J = 16 Hz, 2-CH); 4.13 (1H, d, J = 16 Hz, 2-CH); 7.12 - 7.15 (1H, m, 4-CH).

## EXAMPLE 4

3-Methyl-5-(1,5-dimethyl-1,2,5,6-tetrahydropyridin-3-yl)-1,2,4-oxadiazole hydrogen oxalate

To a stirred solution of 3-methyl-5-(5-methyl-1,2,5,6-tetrahydropyridin-3-yl)-1,2,4-oxadiazole (0.335 g, 0.0019 mol, Example 1i) in dry tetrahydrofuran (10 ml), under a nitrogen atmosphere, was added sodium hydride (87 mg, 0.002 mol of a 55% dispersion in oil). After 20 minutes bromomethane (1 ml, 0.0020 mol of a 2M solution in diethyl ether) was added dropwise. After 6 hours further bromomethane (1 ml of a 2M solution) was added and the mixture stirred for a further 6 hours. Water (20 ml) was added followed by dichloromethane (20 ml). After stirring for 10 minutes the organic layer was separated, aqueous re-extracted with dichloromethane (2 x 20 ml). The combined organics were washed with water (20 ml), dried (sodium sulphate) then evaporated to dryness in vacuo to yield a yellow oil (0.31 g) which was purified by column chromatography on silica by elution with dichloromethane/methanol (gradient elution) to afford the title compound free base as a pale yellow oil (115 mg, 32%).

The hydrogen oxalate salt had mp 115 - 120 °C (propan-2-ol/methanol (2:1), Rf = 0.60 in dichloromethane/methanol (9:1) on silica. (Found: C, 48.18, H, 4.78; N, 13.36; $C_{10}H_{15}N_3O.1.25C_2H_2O_4.0.25H_2O$ requires C, 48.38; H, 5.84; N, 13.54%); $\nu$ max (nujol) 3600 - 3200 $cm^{-1}$ (OH of oxalic acid), 2850 - 2400 $cm^{-1}$ ($NH^+$), 1700 $cm^{-1}$ (C = O of oxalic acid), 1640 and 1620 $cm^{-1}$ (C = C, C = N and C = O of oxalic acid); m/e 193 ($M^+$ of free base); $\delta$ (360 MHz, $d_6$DMSO) 1.10 (3H, d, J = 7 Hz, $CHCH_3$); 2.35 (3H, s, oxadiazole-$CH_3$); 2.60 (1H, dd, J = 8, 11 Hz, 6-CH); 2.69 (3H, s, $NCH_3$); 2.75 - 2.85 (1H, m, 5-CH); 3.19 (1H, dd, J = 6, 11 Hz, 6-CH); 3.66 (1H, d, J = 16 Hz, 2-CH); 3.85 (1H, d, J = 16 Hz, 2-CH); 7.01 (1H, broad s, 4-CH).

## EXAMPLE 5

3-Ethyl-5-(1,5-dimethyl-1,2,5,6-tetrahydropyridin-3-yl)-1,2,4-oxadiazole hydrogen oxalate

To a stirred solution of 3-ethyl-5-(5-methyl-1,2,5,6-tetrahydropyridin-3-yl)-1,2,4-oxadiazole (0.46 g, 0.0024 mol, Example 2h) in dry acetone (8 ml) was added iodomethane (0.15 ml, 0.0024 mol) dropwise. After 24 hours the reaction mixture was evaporated and the residue was dissolved in water (15 ml), basified to pH = 10 with potassium carbonate and extracted with dichloromethane (2 x 20 ml). The combined organics were dried (sodium sulphate) then evaporated to dryness in vacuo to give a pale yellow oil (0.485 g) which was purified by column chromatography on silica by elution with dichloromethane/methanol (gradient elution) to afford the title product free base as a pale yellow oil (100 mg, 20%). The hydrogen oxalate salt had mp 109 -110 °C (propan-2-ol/diethyl ether), Rf = 0.75 in dichloromethane/methanol (9:1) on silca. (Found, C, 51.35; H, 6.50; N, 13.72; $C_{11}H_{17}N_3O.\ C_2H_2O_4.0.5H_2O$ requires C, 50.97; H, 6.58; N, 13.72%); m/e 207 ($M^+$ of free base); $\delta$ (360 MHz, $D_2O$) 1.21 (3H, d, J = 7 Hz, $CHCH_3$); 1.28 (3H, t, J = 7.5 Hz, $CH_2CH_3$); 2.78 (2H, q, J = 7.5 Hz, $CH_2CH_3$); 2.94 - 3.00 (2H, m, 5-CH and 6-CH); 3.09 (3H, s, $NCH_3$); 3.68 - 3.72 (1H, m, 6-CH); 4.03 (1H, dm, J = 16 Hz, 2-CH); 4.34 (1H, d, J = 16 Hz, 2-CH); 7.18 (1H, broad s, 4-CH).

## EXAMPLE 6

3-Dimethylamino-5-(1,5-dimethyl-1,2,5,6-tetrahydropyridin-3-yl)-1,2,4-oxadiazole sesquioxalate

The title compound free base was obtained as a pale yellow oil (65 mg, 15%) from 3-dimethylamino-5-(5-methyl-1,2,5,6-tetrahydropyridin-3-yl)-1,2,4- oxadiazole (0.41 g, 0.0020 mol, Example 3c) and iodomethane (0.12 ml, 0.0020 mol) by the procedure described for Example 5. The sesquioxalate salt had mp 138 - 140 °C (propan-2-ol/diethyl ether), Rf = 0.67 in dichloromethane/methanol (9:1) on silica. (Found: C, 46.92;; H, 5.89; N, 15.61; $C_{11}H_{18}N_4O$. $1.5C_2H_2O_4$ requires C, 47.06; H, 5.92; N, 15.68%); m/e 224 $(M+2H)^+$ of free base; $\delta$ (360MHz, $D_2O$) 1.19 (3H, d, J = 7 Hz, $CHCH_3$); 2.92 - 3.04 (2H, m, 5-CH and 6-CH); 2.98 (9H, s, $N(CH_3)_2$ and $NCH_3$); 3.64 - 3.72 (1H, m, 6-CH); 3.97 (1H, dm, J = 16 Hz, 2-CH); 4.32 (1H, d, J = 16 Hz, 2-CH); 7.10 (1H, broad s,4-CH).

## EXAMPLE 7

### Tablet Preparation

Tablets containing 1.0, 2.0, 25.0, 26.0, 50.0 and 100.0 mg., respectively, of the following compound are prepared as illustrated below:
3-methyl-5-(5-methyl-1,2,5,6-tetrahydropyrid-3-yl)-1,2,4-oxadiazole.

| TABLE FOR DOSES CONTAINING FROM 1-25 MG OF THE ACTIVE COMPOUND | | | |
|---|---|---|---|
| | Amount-mg | | |
| Active Compound | 1.0 | 2.0 | 25.0 |
| Microcrystalline cellulose | 49.25 | 48.75 | 37.25 |
| Modified food corn starch | 49.25 | 48.75 | 37.25 |
| Magnesium stearate | 0.50 | 0.50 | 0.50 |
| **TABLE FOR DOSES CONTAINING FROM 26-100 MG OF THE ACTIVE COMPOUND** | | | |
| | Amount-mg | | |
| Active Compound | 26.0 | 50.0 | 100.0 |
| Microcrystalline cellulose | 52.05 | 100.0 | 200.0 |
| Modified food corn starch | 2.21 | 4.25 | 8.5 |
| Magnesium stearate | 0.39 | 0.75 | 1.5 |

All of the active compound, lactose, and a portion of the corn starch are mixed and granulated to a 10% corn starch paste. The resulting granulation is sieved, dried and blended with the remainder of the corn starch and the magnesium stearate. The resulting granulation is then compressed into tablets containing 1.0 mg, 2.0 mg, 25.0 mg, 26.0 mg, 50.0 mg, and 100.0 mg of active ingredient per tablet.

## Claims

1. A compound of formula I, or a salt or prodrug thereof:

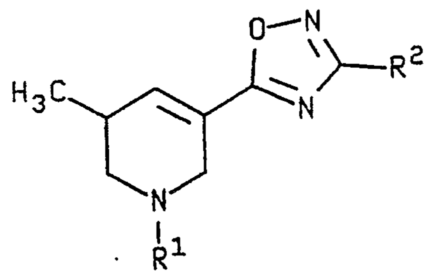

(I)

wherein $R^1$ represents hydrogen or methyl; and
$R^2$ represents hydrogen, methyl, ethyl, ethenyl, amino, methylamino, dimethylamino, chloro, bromo, cyano or methoxy.

2. A compound as claimed in claim 1 wherein $R^2$ is methyl, ethyl or dimethylamino.

3. A compound as claimed in claim 1 wherein $R^1$ represents hydrogen or methyl; and $R^2$ represents ethenyl, amino, methylamino, dimethylamino, chloro, bromo, cyano or methoxy.

4. A compound as claimed in claim 1 selected from:
3-methyl-5-(5-methyl-1,2,5,6-tetrahydropyrid-3-yl)-1,2,4-oxadiazole;
3-methyl-5-(1,5-dimethyl-1,2,5,6-tetrahydropyrid-3-yl)-1,2,4-oxadiazole;
3-ethyl-5-(1,5-dimethyl-1,2,5,6-tetrahydropyrid-3-yl)-1,2,4-oxadiazole;
3-dimethylamino-5-(5-methyl-1,2,5,6-tetrahydropyrid-3-yl)-1,2,4-oxadiazole;
3-dimethylamino-5-(1,5-dimethyl-1,2,5,6-tetrahydropyrid-3-yl)-1,2,4-oxadiazole;
3-amino-5-(5-methyl-1,2,5,6-tetrahydropyrid-3-yl)-1,2,4-oxadiazole;
3-amino-5-(1,5-dimethyl-1,2,5,6-tetrahydropyrid-3-yl)-1,2,4-oxadiazole;
3-cyano-5-(5-methyl-1,2,5,6-tetrahydropyrid-3-yl)-1,2,4-oxadiazole;
3-cyano-5-(1,5-dimethyl-1,2,5,6-tetrahydropyrid-3-yl)-1,2,4-oxadiazole;
3-ethenyl-5-(5-methyl-1,2,5,6-tetrahydropyrid-3-yl)-1,2,4-oxadiazole;
3-ethenyl-5-(1,5-dimethyl-1,2,5,6-tetrahydropyrid-3-yl)-1,2,4-oxadiazole;
3-methoxy-5-(5-methyl-1,2,5,6-tetrahydropyrid-3-yl)-1,2,4-oxadiazole;
3-methoxy-5-(1,5-dimethyl-1,2,5,6-tetrahydropyrid-3-yl)-1,2,4-oxadiazole;
3-methylamino-5-(5-methyl-1,2,5,6-tetrahydropyrid-3-yl)-1,2,4-oxadiazole;
3-methylamino-5-(1,5-dimethyl-1,2,5,6-tetrahydropyrid-3-yl)-1,2,4-oxadiazole;
and salts and prodrugs thereof.

5. A pharmaceutical composition comprising a compound as claimed in any one of the preceding claims in association with a pharmaceutically acceptable carrier or excipient.

6. A compound as claimed in any one of claims 1 to 4 for use as a therapeutic agent.

7. The use of a compound as claimed in any one of claims 1 to 4 for the preparation of a medicament for the treatment and/or prevention of neurological and mental disorders and/or of severe painful conditions.

8. A process for the preparation of a compound as claimed in any one of claims 1 to 4 which comprises reacting a reactive derivative of a carboxylic acid of formula II:

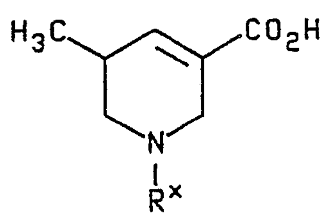

(II)

wherein $R^x$ represents the group $R^1$ as defined in claim 1 or an N-protecting group; with a compound of formula III:

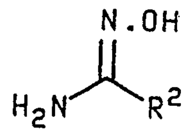

(III)

wherein $R^2$ is as defined in claim 1; and subsequently, if necessary, removing the N-protecting group; followed, if desired, by methylation of the compound of formula I wherein $R^1$ represents hydrogen thereby obtained.

Claims for the following Contracting State : ES

1. A process for the preparation of a compound of formula I, or a salt or prodrug thereof:

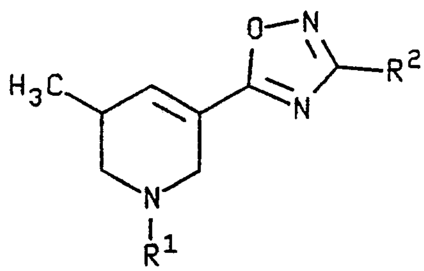

(I)

wherein $R^1$ represents hydrogen or methyl; and
$R^2$ represents hydrogen, methyl, ethyl, ethenyl, amino, methylamino, dimethylamino, chloro, bromo, cyano or methoxy; which process comprises reacting a reactive derivative of a carboxylic acid of formula II:

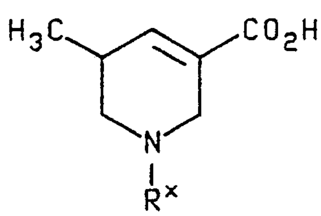

(II)

wherein $R^x$ represents the group $R^1$ as defined above or an N-protecting group; with a compound of formula III:

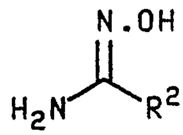

(III)

wherein $R^2$ is as defined above; and subsequently, if necessary, removing the N-protecting group; followed, if desired, by methylation of the compound of formula I wherein $R^1$ represents hydrogen thereby obtained.

2. A process as claimed in claim 1 for the preparation of a compound wherein $R^2$ is methyl, ethyl or dimethylamino.

3. A process as claimed in claim 1 for the preparation of a compound wherein $R^1$ represents hydrogen or methyl; and $R^2$ represents ethenyl, amino, methylamino, dimethylamino, chloro, bromo, cyano or methoxy.

4. A process as claimed in claim 1 for the preparation of a compound selected from:
3-methyl-5-(5-methyl-1,2,5,6-tetrahdropyrid-3-yl)-1,2,4-oxadiazole;
3-methyl-5-(1,5-dimethyl-1,2,5,6-tetrahdropyrid-3-yl)-1,2,4-oxadiazole;
3-ethyl-5-(1,5-dimethyl-1,2,5,6-tetrahydropyrid-3-yl)-1,2,4-oxadiazole;
3-dimethylamino-5-(5-methyl-1,2,5,6-tetrahydropyrid-3-yl)-1,2,4-oxadiazole;
3-dimethylamino-5-(1,5-dimethyl-1,2,5,6-tetrahydropyrid-3-yl)-1,2,4-oxadiazole;
3-amino-5-(5-dimethyl-1,2,5,6-tetrahydropyrid-3-yl)-1,2,4-oxadiazole;
3-amino-5-(1,5-dimethyl-1,2,5,6-tetrahydropyrid-3-yl)-1,2,4-oxadiazole;
3-cyano-5-(5-methyl-1,2,5,6-tetrahydropyrid-3-yl)-1,2,4-oxadiazole;
3-cyano-5-(1,5-dimethyl-1,2,5,6-tetrahydropyrid-3-yl)-1,2,4-oxadiazole;
3-ethenyl-5-(5-methyl-1,2,5,6-tetrahydropyrid-3-yl)-1,2,4-oxadiazole;
3-ethenyl-5-(1,5-dimethyl-1,2,5,6-tetrahydropyrid-3-yl)-1,2,4-oxadiazole;
3-methoxy-5-(5-methyl-1,2,5,6-tetrahydropyrid-3-yl)-1,2,4-oxadiazole;
3-methoxy-5-(1,5-dimethyl-1,2,5,6-tetrahydropyrid-3-yl)-1,2,4-oxadiazole;
3-methylamino-5-(5-methyl-1,2,5,6-tetrahydropyrid-3-yl)-1,2,4-oxadiazole;
3-methylamino-5-(1,5-dimethyl-1,2,5,6-tetrahydropyrid-3-yl)-1,2,4-oxadiazole;
and salts and prodrugs thereof.

5. A process for the preparation of a pharmaceutical composition which comprises mixing a compound prepared as claimed in any one of the preceding claims with a pharmaceutically acceptable carrier or excipient.

6. The use of a compound prepared as claimed in any one of claims 1 to 4 for the preparation of a medicament for the treatment and/or prevention of neurological and mental disorders and/or of severe painful conditions.